# EUROPÄISCHE PATENTSCHRIFT

(11) **EP 1 660 482 B1**
(45) Veröffentlichungstag und Bekanntmachung des Hinweises auf die Patenterteilung: **29.04.2009**
(21) Anmeldenummer: 04764411.7
(22) Anmeldetag: 24.08.2004
(51) Int. Cl.: C07D 401/12, A61K 31/4439, A61P 7/02

(54) **3-[(2-{ 4-(HEXYLOXYCARBONYLAMINO-IMINO-METHYL)-PHENYLAMINO]-METHYL}-1-METHYL-1H-BENZIMIDAZOL-5-CARBONYL)-PYRIDIN-2-YL-AMINO] -PROPIONSÄURE-ETHYLESTER -METHANSULFONAT UND DESSEN VERWENDUNG ALS ARZNEIMITTEL**
3-[(2-{ 4-(HEXYLOXYCARBONYLAMINO-IMINO-METHYL)-PHENYLAMINO] -METHYL}-1-METHYL-1H-BENZIMIDAZOL-5-CARBONYL)-PYRIDIN-2-YL-AMINO -PROPIONIC ACID ETHYL ESTER METHANE SULPHONATE AND USE THEREOF AS A MEDICAMENT
ETHYLESTER-METHANSULFONATE D'ACIDE 3-[(2-{[4-(HEXYLOXYCARBONYLAMINO-IMINO-METHYL)-PHENYLAMINO]-METHYL}-1-METHIL-1H - -BENZIMIDAZOL-5-CARBONYL)-PYRIDIN-2-YL-AMINO]-PROPIONIQUE ET SON UTILISATION EN TANT QUE MEDICAMENT

(30) Priorität: 29.08.2003 DE 10339862
(43) Veröffentlichungstag der Anmeldung: 31.05.2006
(60) Teilanmeldung: 09153155.8
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: SOBOTTA, Rainer, 55218 INGELHEIM (DE); SIEGER, Peter, 88441 MITTELBIBERACH (DE); SCHMID, Rolf, 88487 BALTRINGEN (DE)
(86) Internationale Anmeldenummer: PCT/EP2004/009432
(87) Internationale Veröffentlichungsnummer: WO 2005/028468

(56) Entgegenhaltungen:
- DE-A- 10 245 624
- HAUEL N H ET AL: "STRUCTURE-BASED DESIGN OF NOVEL POTENT NONPEPTIDE THROMBIN INHIBITORS" JOURNAL OF MEDICINAL CHEMISTRY, AMERICAN CHEMICAL SOCIETY. WASHINGTON, US, Bd. 45, Nr. 9, 2002, Seiten 1757-1766, XP001098844 ISSN: 0022-2623
- MUNGALL D: "BIBR-1048 BOEHRINGER INGELHEIM" CURRENT OPINION IN INVESTIGATIONAL DRUGS, PHARMAPRESS, US, Bd. 3, Nr. 6, Juni 2002 (2002-06), Seiten 905-907, XP001147306 ISSN: 1472-4472
- CAIRA M R: "CRYSTALLINE POLYMORPHISM OF ORGANIC COMPOUNDS" TOPICS IN CURRENT CHEMISTRY, SPRINGER, BERLIN, DE, Bd. 198, 1998, Seiten 163-208, XP001156954 ISSN: 0340-1022
- COLLINS B.; HOLLIDGE C.: 'Antithrombotic drug market' NATURE REVIEWS DRUG DISCOVERY 2003, Seiten 11 - 12

## Beschreibung

Gegenstand der vorliegenden Erfindung ist die Verbindung 3-[(2-{[4-(Hexyloxy carbonylamino-imino-methyl)-phenylamino]-methyl}-methyl-1*H*-benzimidazol-5-carbonyl)-pyridin-2-yl-amino]-propionsäure-ethylester - Methansulfonat der Formel A und deren Verwendung als Arzneimittel.

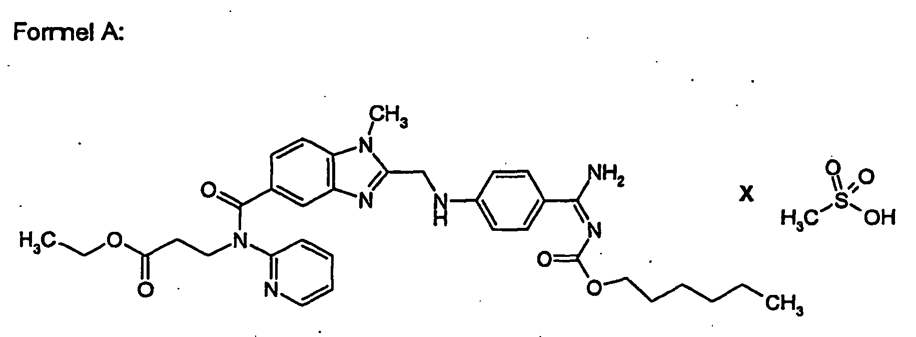

Die Base der Verbindung der Formel Ä ist bereits aus der WO 98/37075, in der Verbindungen mit einer Thrombin-hemmenden und die Thrombinzeit verlängernden Wirkung offenbart werden, unter dem Namen 1-Methyl-2-[*N*-[4-(*N*-n-hexyloxycarbonyl-amidino)phenA-amino-methyq-benzimidazol-5-yl-carbonsäure-*N*-(2-pyridyl)-*N*-(2-ethoxycarbonylethyl)-amid bekannt. Bei der Verbindung der Formel A handelt es sich um ein Doppel-Prodrug der Verbindung 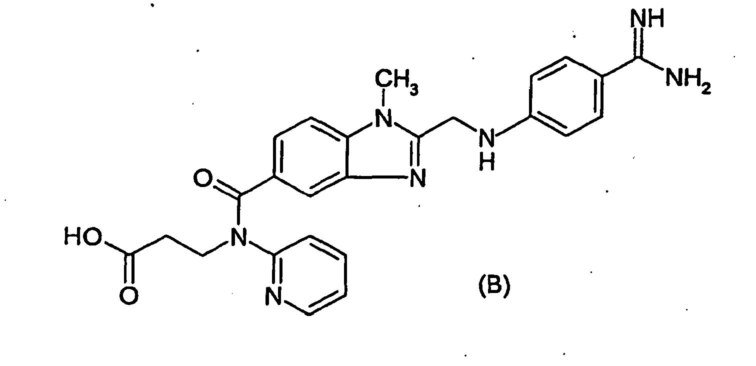 d.h. die Verbindung der Formel A (BIBR 1048 MS) wird erst im Körper in die eigentlich wirksame Verbindung, nämlich die Verbindung der Formel B, umgewandelt. Hauptindikationsgebiete der Verbindung der chemischen Formel A sind die post-operative Prophylaxe von tiefen Venenthrombosen und die Prophylaxe von Sch laganfällen.

Die vorstehend genannten pharmakologisch wertvollen Eigenschaften der im Stand der Technik offenbarten disubstituierten bicyclischen Heterocyclen stellen die Grundvoraussetzung für eine wirksame Verwendung der Verbindungen als Arzneimittel dar. Ein Wirkstoff muß allerdings noch weiteren Anforderungen gerecht werden, um als Arzneimittel zum Einsatz gelangen zu können. Diese Parameter sind zu einem großen Teil mit der physikochemischen Beschaffenheit des Wirkstoffs verbunden.

Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der Arzneimitelzusammensetzungen verwendete Arzneiwirkstoff sollte daher eine hohe Stabilität aufweisen, die auch unter verschiedenen Umgebungsbedingungen gewährleistet sein muß. Dies ist zwingend erforderlich, um zu verhindern, dass Arzneimittelzusammensetzungen Verwendung finden, in denen neben tatsächlichem Wirkstoff beispielsweise Abbauprodukte desselben enthalten sind. In einem solchen Fall könnte ein in pharmazeutischen Formulierungen vorgefundener Gehalt an Wirkstoff niedriger sein als spezifiziert.

Die Absorption von Feuchtigkeit vermindert den Gehalt an Arzneiwirkstoff wege n der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird. Vorzugsweise sollte ein Arzneimittetwirkstoff daher nur in geringem Maße hygroskopisch sein.

Da die Kristallmodifikation eines Wirkstoffs für den reproduzierbaren Wirkstoffgehalt Da die Kristallmodifikation eines Wirkstoffs für den reproduzierbaren Wirkstoffgehalt einer Darreichungsform von Bedeutung ist, besteht die Notwendigkeit, eventuell existierenden Polymorphismus eines kristallin vorliegenden Wirkstoffs bestmöglich aufzuklären. Sofern verschiedene polymorphe Modifikationen eines Wirkstoffs auftreten, sollte gewährleistet sein, dass sich, die kristalline Modifikation der Substanz in der späteren Arzneimittelzubereitung nicht verändert. Andernfalls könnte dies die reproduzierbare Wirksamkeit des Medikaments nachteilig beeinflussen. Bevorzugt sind vor diesem Hintergrund Wirkstoffe, die nur durch geringen Polymorphismus gekennzeichnet sind.

Ein weiteres Kriterium, welches je nach Wahl der Formulierung oder nach Wahl des Herstellungsverfahrens der Formulierung von unter Umständen herausragender Bedeutung ist, ist die Löslichkeit des Wirkstoffs. Werden beispielsweise Arzneimittellösungen (etwa für Infusionen) bereitgestellt, so ist eine ausreichende Löslichkeit des Wirkstoffs in physiologisch verträglichen Lösemitteln unverzichtbar. Auch für oral zu applizierende Arzneimittel ist eine ausreichende Löslichkeit des Wirkstoffs von großer Richtigkeit.

Der vorliegenden Erfindung liegt die Aufgabe zugrunde, einen Arzneimittelwirkstoff bereitzustellen, der nicht nur durch eine hohe pharmakologische Wirksamkeit gekennzeichnet ist, sondern ferner den vorstehend genannten physikochemischen Anforderungen bestmöglich gerecht wird.

### Detaillierte Beschreibung der Erfindung

Die vorstehend genannte Aufgabe wird durch das Salz 3-[(2-{[4-(Hexyloxycarbonylamino-imino-methyl)-phenylamino]-methyl}-methyl-1*H*-benzimidazol-5-carbonyl)-pyridin-2-yl-amino]-propionsäure-ethylester-Methansulfonat (Form II) der Formel A gelöst.

Überraschend wurde nämlich gefunden, dass nach den in den Beispielen 1 bis 3 beschriebenen Verfahren die Kristallmodifikation II dieses Salzes jeweils selektiv und einheitlich hergestellt werden kann.

Für die Arzneimittelanwendung ist es erforderlich, dass der darin enthaltene Wirkstoff in einer einheitlichen Kristallmodifikation vorliegt, damit eine zuverlässige Bioverfügbarkeit gewährleistet ist.

Das erfindungsgemäße Methansulfonat zeichnet sich in allen drei Kristallmodifikationen durch gute Kristallinität und eine geringe Amorphisierung beim Vermahlen und Verpressen aus. Es ist zudem in allen drei Kristallmodifikatfonen nicht hygroskopisch und in physiologisch verträglichen sauren wäßrigen Medien sehr gut löslich.

Die kristallinen Formen des erfindungsgemäßen Methansulfonats der Verbindung 3-[(2-{[4-(Hexyloxycarbonylamino-imino-methyl)-phenylamlno]-methyl}-1-methyl-1*H-*benzimidazol-5-carbonyl)-pyridin-2-yl-amino]-propionsäure-ethylester sind gekennzeichnet durch einen Schmelzpunkt von T_{Smp.} = 180 ± 3°C (Form I), T_{Smp.} =190 ± 3°C (Form II) bzw. T_{Smp.} = 120 ± 5°C (Hemihydrat) (bestimmt über DSC = Differential Scanning Calorimetry; Auswertung über Peak-Maximum; Heizrate: 10°C/min). Die aufgeführten Werte wurden mittels eines DSC 821^{e} der Firma Mettler Toledo ermittelt.

Ein erster Gegenstand der vorliegenden Erfindung ist daher die oben erwähnte polymorphe Form (II) des Salzes 3-[(2-{[4-(Hexyloxycarbonylamino-imino-methyl)-phenylamino]-methyl}-1-methyl-1*H*-benzlmidazol-5-carbonyl)-pyridin-2-ylamino]-propionsäure-ethylester- Methansulfonat, bevorzugt in kristalliner Form, gekennzeichnet durch Schmelzpunkte von T_{Smp.} = 190 ± 3°C (bestimmt über DSC; Auswertung über Peak-Maximum; Heizrate: 10°C/min).

Ein weiterer Erfindungsgegenstand sind die Verfahren zur selektiven Herstellung der polymorphen Form (II) sowie die nach diesem Verfahren erhältlichen Modifikation.

Man erhält BIBR 1048 MS Polymorph I, indem man
a) eine Lösung von BIBR 1048 Base in Aceton bei einer Temperatur von ca. 30 °C bis 36 °C langsam mit einer Lösung von einem geringen Unterschuß (beispielsweise 0,98 Äquivalente) Methansulfonsäure in Aceton versetzt,
b) bei einer Temperatur von ca. 26 °C bis 33 °C etwa 1 Stunde nachrührt,
c) auf ca. 17 °C bis 23 °C abkühlt und weitere 40 bis 80 Minuten lang bei dieser Temperatur nachrührt,
d) die ausgefallenen Kristalle von BIBR 1048 MS Form I absaugt und
e) das so erhaltene Produkt mindestens 4 Stunden bei maximal 50 °C im Vakuum trocknet.

Erfindungsgemäß erhält man BIBR 1048 MS Polymorph II, indem man
a) eine Lösung von BIBR 1048 Base in Aceton bei einer Temperatur von ca. 40 °C bis 46 °C langsam mit einer Lösung von einem geringen Unterschuß (beispielsweise 0,98 Äquivalente) Methansulfonsäure in Aceton versetzt,
b) gegebenenfalls mit BIBR 1048 Polymorph II Kristallen animpft.
c) bei einer Temperatur von ca. 40 °C bis 46 °C etwa 1 Stunde nachrührt,
d) auf ca. 17 °C bis 23 °C abkühlt und weitere 40 bis 80 Minuten lang bei dieser Temperatur nachrührt,
e) die ausgefallenen Kristalle von BIBR 1048 MS Form II absaugt und
f) das so erhaltene Produkt mindestens 4 Stunden bei maximal 50 °C im Vakuum trocknet;
oder indem man
a) eine Suspension von BIBR 1048 MS Polymorph I in Aceton unter Rühren ca. 4 Stunden lang auf 45 °C bis 50 °C erwärmt,
b) gegebenenfalls
   i) mit BIBR 1048 Polymorph II Kristallen animpft, oder
   ii) mit BIBR 1048 Polymorph II Kristallen animpft und zusätzlich eine geringe Menge BIBR 1048 Base zugibt,
c) dann auf ca. 15 °C abkühlt,
d) die ausgefallenen Kristalle von BIBR 1048 MS Form II absaugt und
e) das so erhaltene Produkt mindestens 4 Stunden bei maximal 50 °C im Vakuum trocknet;
oder indem man
a) BIBR 1048 MS Polymorph I in Aceton vorlegt und
b) gegebenenfalls
   i) mit einer geringen Menge BIBR 1048 Polymorph II animpft, oder
   ii) mit BIBR 1048 Polymorph II Kristallen animpft und zusätzlich eine geringe Menge BIBR 1048 Base zugibt,
c) die so erhalten Mischung unter Rühren mindestens eine Stunde lang auf 40 °C bis 46 °C erwärmt,
d) dann auf ca. 17 °C bis 23 °C abkühlt und weitere 40 bis 80 Minuten lang bei dieser Temperatur nachrührt,
e) die ausgefallenen Kristalle von BIBR 1048 MS Form II abtrennt und
f) das so erhaltene Produkt mindestens 4 Stunden bei maximal 50 °C im Vakuum trocknet.

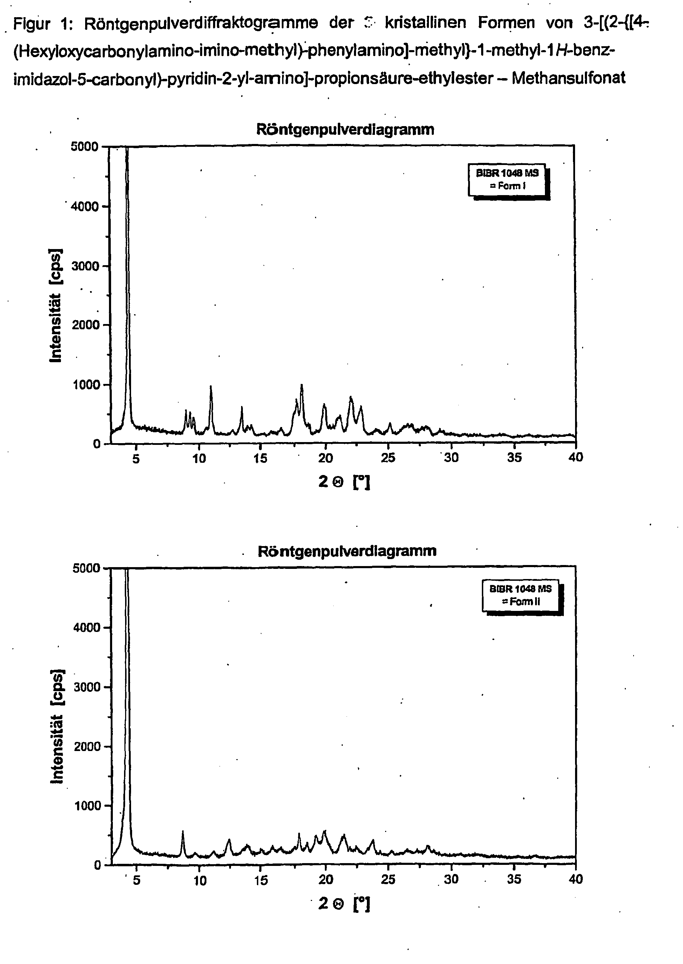

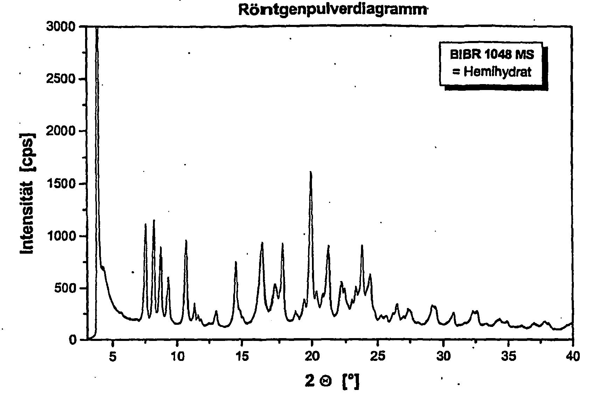

Die erfindungsgemäße kristalline Form des 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Methansulfonats wurden mittels Röntgenpulverbeugung näher untersucht. Die erhaltenen Diagramme sind in Figur 1 dargestellt.

Die nachstehenden Tabellen 1 bis 3 führen die bei dieser Analyse erhaltenen Daten auf:

**Tabelle 1 (Referenz): Röntgenpulverreflexe und Intensitäten (normiert) des 3-[(2-{[4-(Hexyloxycarbonylamino-imino-methyl)-phenylamino]-methyl}-methyl-1H-benzimldazol-5-carbonyl)-pyridin-2-yl-amino]-propionsäure-ethylester - Methansulfonats (Form I)**

| **2 Θ** **[°]** | **dₕₖₗ-Wert** **[Å]** | **Intensität [%]** |
|---|---|---|
| 4,4 | 20,1 | 100 |
| 8,94 | 9,90 | 5 |
| 9,23 | 9,57 | 4 |
| 9,55 | 9,26 | 4 |
| 10,55 | 8,38 | 2 |
| 10,95 | 8,08 | 11 |
| 12,73 | 6,95 | 1 |
| 13,46 | 6,57 | 7 |
| 13,95 | 6,34 | 3 |
| 14,26 | 6,21 | 2 |
| 15,17 | 5,84 | 1 |
| 15,93 | 5,56 | 1 |
| 16,46 | 5,38 | 1 |
| 17,66 | 5,02 | 8 |
| 18,07 | 4,91 | 13 |
| 18,60 | 4,77 | 2 |
| 19,89 | 4,46 | 6 |
| 20,28 | 4,38 | 2 |
| 20,54 | 4,32 | 2 |
| 21,12 | 4,20 | 4 |
| 22,06 | 4,03 | 8 |
| 22,85 | 3,89 | 6 |
| 24,12 | 3,69 | 1 |
| 25,10 | 3,54 | 3 |
| 25,99 | 3,43 | 1 |
| 26,52 | 3,36 | 2 |
| 26,83 | 3,32 | 2 |
| 27,16 | 3,28 | 1 |
| 27,64 | 3,22 | 2 |
| 28,09 | 3,17 | 2 |
| 29,08 | 3,07 | 1 |
| 29,26 | 3,05 | 1 |
| 29,94 | 2,98 | 1 |
| 31,88 | 2,80 | 1 |
| 34,37 | 2,61 | 1 |
| 36,21 | 2,48 | 1 |
| 38,26 | 2,35 | 1 |
| 39,47 | 2,28 | 1 |
| 39,98 | 2,25 | 1 |

**Tabelle 2: Röntgenpulverreflexe und Intensitäten (normiert) des 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester - Methansulfonats (Form II)**

| **2 Θ** **[°]** | **dₕₖₗ-Wert** **[Å]** | **Intensität [%]** |
|---|---|---|
| 4,3 | 20,4 | 100 |
| 8,72 | 10,1 | 3 |
| 9,68 | 9,13 | 1 |
| 11,15 | 7,93 | 1 |
| 12,42 | 7,12 | 2 |
| 13,59 | 6,51 | 1 |
| 13,95 | 6,34 | 1 |
| 15,11 | 5,86 | 1 |
| 15,97 | 5,55 | 1 |
| 16,52 | 5,36 | 1 |
| 17,45 | 5,08 | 1 |
| 17,86 | 4,96 | 2 |
| 18,45 | 4,81 | 1 |
| 19,22 | 4,61 | 2 |
| 19,89 | 4,46 | 2 |
| 21,46 | 4,14 | 2 |
| 21,98 | 4,04 | 1 |
| 22,48 | 3,95 | 1 |
| 23,75 | 3,74 | 1 |
| 25,29 | 3,52 | 1 |
| 28,17 | 3,17 | 1 |
| 28,59 | 3,12 | 1 |

**Tabelle 3 (Referenz): Röntgenpulverreflexe und Intensitäten (normiert) des 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester - Methansulfonats (Hemihydrat)**

| **2 Θ** **[°]** | **dₕₖₗ-Wert** **[Å]** | **Intensität [%]** |
|---|---|---|
| 3,9 | 22,8 | 100 |
| 4,4 | 20,1 | 10 |
| 5,64 | 15,7 | 2 |
| 7,57 | 11,8 | 16 |
| 8,25 | 10,7 | 17 |
| 8,77 | 10,1 | 12 |
| 9,34 | 9,46 | 7 |
| 10,69 | 8,27 | 13 |
| 11,33 | 7,80 | 3 |
| 11,66 | 7,58 | 1 |
| 11,96 | 7,39 | 1 |
| 13,04 | 6,78 | 3 |
| 14,54 | 6,09 | 11 |
| 15,16 | 5,84 | 1 |
| 16,56 | 5,35 | 13 |
| 17,27 | 5,13 | 6 |
| 17,78 | 4,98 | 12 |
| 18,75 | 4,73 | 1 |
| 19,41 | 4,57 | 3 |
| 19,95 | 4,45 | 24 |
| 20,38 | 4,35 | 4 |
| 20,84 | 4,26 | 4 |
| 21,21 | 4,19 | 12 |
| 22,22 | 4,00 | 6 |
| 22,46 | 3,96 | 5 |
| 23,05 | 3,85 | 3 |
| 23,40 | 3,80 | 4 |
| 23,85 | 3,73 | 12 |
| 24,44 | 3,64 | 7 |
| 25,30 | 3,52 | 1 |
| 25,63 | 3,47 | 1 |
| 26,22 | 3,40 | 2 |
| 26,52 | 3,36 | 3 |
| 27,06 | 3,29 | 1 |
| 27,45 | 3,25 | 2 |
| 29,27 | 3,05 | 3 |
| 30,78 | 2,90 | 2 |
| 32,32 | 2,77 | 2 |
| 32,59 | 2,75 | 2 |
| 34,31 | 2,61 | 1 |
| 34,91 | 2,57 | 1 |
| 36,04 | 2,49 | 1 |
| 37,00 | 2,43 | 1 |
| 37,84 | 2,38 | 1 |
| 38,13 | 2,36 | 1 |

In den voranstehenden Tabellen 1 bis 3 steht der Wert "2 Θ [°]" für den Beugungswinkel in Grad und der Wert "dₕₖₗ [Å]" für die bestimmten Abstände in Å zwischen den Gitterebenen.

Die Röntgenpulverdiagramme wurden im Rahmen der vorliegenden Erfindung mittels eines Bruker D8 Advanced - Diffraktometers, ausgerüstet mit einem ortsempfindlichen Detektor (OED) und einer Cu-Anode als Röntgenquelle (CuK_{α} - Strahlung, λ = 1.54056 Å, 40 kV, 40 mA) aufgenommen.

Das Hydrat der Verbindung 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester -Methansulfonat liegt unter Normbedingungen in Form des Hemihydrats vor, aus dem bei einer Temperatur von etwa 120°C, parallel zum Aufschmelzen dieser Form, Wasser entweicht.

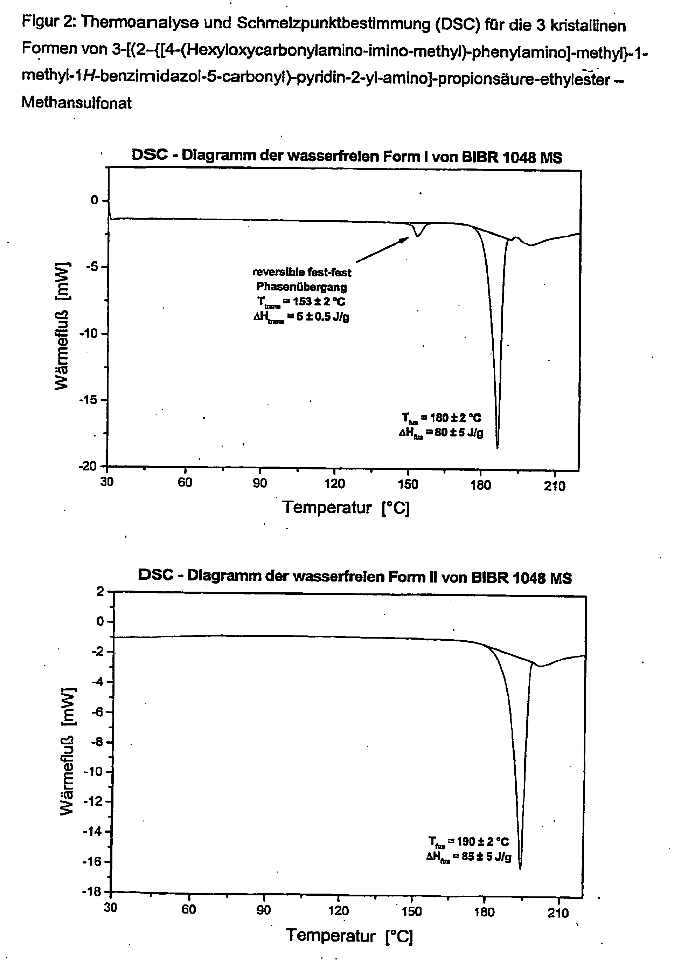

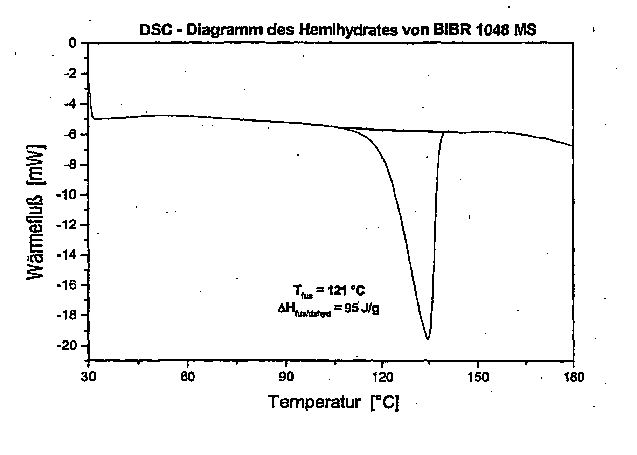

Figur 2 gibt die Thermoanalyse der drei Formen wieder.

### Experimenteller Teil

### Referenzbeispiel

### 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester- Methansulfonat Form I (BIBR 1048 MS Polymorph I)

In einen inertisieren Rührwerksapparat werden 52,6 kg 3-[(2-{[4-(Hexyloxycarbonylamino-imino-methyl)-phenylamino]-methyl}-1-methy/-1*H*-benzimidazol-5-carbonyl)-pyridin-2-yl-amino]-propionsäure-ethylester - Base (die bevorzugterweise zuvor durch Umkristallisation aus Essigsäureethylester gereinigt wurde) vorgelegt und anschließend 293 kg Aceton eingezogen. Der Apparateinhalt wird unter Rühren auf 40 bis 46°C erwärmt. Nachdem eine klare Lösung entstanden ist, wird der Apparateinhalt über ein Linsenfilter in einen zweiten Rührwerksapparat filtriert und anschließend auf 30 bis 36°C abgekühlt. In das Hängegefäß des zweiten Apparates werden nacheinander 33 kg auf 0 bis 5°C vorgekühltes Aceton, 7,9 kg Methansulfonsäure 99,5%ig und zum Nachspülen weitere 9 kg Aceton eingezogen. Der Inhalt des Hängegefäßes wird bei 26 bis 36°C innerhalb von 15 bis 40 Minuten zur Lösung der 3-[(2-{[4-(Hexyloxycarbonylamino-imino-methyl)-phenylamino]-methyl}-1-methyl-1*H-*benzimidazol-5-carbonyl)-pyridin-2-yl-amino]-propionsäure-ethylester - Base zudosiert. Anschließend wird 40 bis 60 Minuten bei 26 bis 33°C nachgerührt. Danach wird auf 17 bis 23°C abgekühlt und weitere 40 bis 80 Minuten gerührt. Die Kristallsuspension wird über einen Filtertrockner abfiltriert und mit insgesamt 270 I Aceton nachgewaschen. Das Produkt wird bei maximal 50°C mindestens 4 Stunden im Vakuum getrocknet.

Ausbeute: 54,5 - 59,4 kg; 90 - 98% d.Th. bez. auf 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base

### Beispiel 1

### BIBR 1048 MS Polymorph II durch Umwandlung aus BIBR 1048 MS Polymorph I

In einen Glaskolben mit Rührer und Rückflusskühler werden 4g BIBR 1048 MS Polymorph I und 35 ml Aceton vorgelegt. Die Suspension wird unter Rühren auf 45 bis 50°C erwärmt und 4 Stunden bei dieser Temperatur gehalten. Anschließend wird auf 15°C abgekühlt und das Kristallisat Ober einen Büchnertrichter abgesaugt, mit 20 ml Aceton nachgewaschen und bei 45°C im Vakuum getrocknet.

Anmerkung: Die Synthese kann auch mit Animpfen mit BIBR 1048 MS Polymorph II durchgeführt werden. Bei geringer Umwandlungsgeschwindigkeit kann diese zusätzlich zum Animpfen mit BIBR 1048 MS Polymorph II durch Zugabe einer geringen Menge BIBR 1048 Base (beispielsweise im technischen Maßstab etwa 50 g BIBR 1048 Base auf ungefähr 90 kg BIBR 1048 MS Polymorph I) beschleunigt werden.

### Beispiel 2

### 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester - Methansulfonat Form II (BIBR 1048 MS Polymorph II)

In einen inertisierten Rührwerksapparat werden 52,6 kg 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base (die bevorzugterweise zuvor durch Umkristallisation aus Essigsäureethylester gereinigt wurde) vorgelegt und anschließend 293 kg Aceton eingezogen. Der Apparateinhalt wird unter Rühren auf 40 bis 46°C erwärmt. Nachdem eine klare Lösung entstanden ist, wird der Apparateinhalt über ein Linsenfilter in einen zweiten Rührwerksapparat filtriert. In das Hängegefäß des zweiten Apparates werden nacheinander 33 kg auf 0 bis 5°C vorgekühltes Aceton, 7,9 kg Methansulfonsäure 99,5%ig und zum Nachspülen weitere 9 kg Aceton eingezogen. Der Inhalt des Hängegefäßes wird bei 40 bis 46°C innerhalb von 15 bis 40 Minuten zur Lösung der 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base zudosiert und mit 10 g BIBR 1048 MS Polymorph II (z.B. hergestellt gemäß Beispiel 2) angeimpft. Anschließend wird 40 bis 60 Minuten bei 40 bis 46°C nachgerührt. Danach wird auf 17 bis 23°C abgekühlt und weitere 40 bis 80 Minuten gerührt. Die Kristallsuspension wird über einen Filtertrockner abfiltriert und mit insgesamt 270 I Aceton nachgewaschen. Das Produkt wird bei maximal 50°C mindestens 4 Stunden im Vakuum getrocknet.

Ausbeute: 54,5 - 59,4 kg; 90 - 98% d.Th. bez. auf 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base

Anmerkung: Die Synthese kann auch ohne Animpfen mit BIBR 1048 MS Polymorph II durchgeführt werden. Bevorzugt ist jedoch das Verfahren mit Animpfen.

### Beispiel 3

### BIBR 1048 MS Polymorph II durch Umwandlung aus BIBR 1048 MS Polymorph I

In einen inertisierten Rührwerksapparat werden 30,7 kg BIBR 1048 MS Polymorph I vorgelegt und anschließend 199 kg Aceton eingezogen. Der Apparateinhalt wird mit 10 g BIBR 1048 MS Polymorph II (z.B. hergestellt gemäß Beispiel 2) angeimpft, unter Rühren auf 40 bis 46°C erwärmt, und mindestens 1 Stunde bei dieser Temperatur gehalten. Danach wird auf 17 bis 23°C abgekühlt und mindestens weitere 40 bis 80 Minuten gerührt.
Die Kristallsuspension wird über eine Stülpzentrifuge abgetrennt und mit insgesamt 45 kg Aceton nachgewaschen. Das Produkt wird bei maximal 50°C mindestens 4 Stunden im Vakuumtrockenschrank getrocknet.
Ausbeute: 27,7 - 30,1 kg; 90 - 98% d.Th.).

Anmerkung: Die Synthese kann auch ohne Animpfen mit BIBR 1048 MS Polymorph II durchgeführt werden. Bevorzugt ist jedoch das Verfahren mit Animpfen. Bei geringer Umwandlungsgeschwindigkeit kann zusätzlich zum Animpfen mit BIBR 1048 MS Polymorph II eine geringe Menge BIBR 1048 Base (beispielsweise etwa 50 g BIBR 1048 Base auf ungefähr 90 kg BIBR 1048 MS Polymorph I) zugegeben werden.

### Kurzbeschreibung der Abbildungen.

Figur 1 zeigt die Röntgenpulverdiffraktogramme der drei kristallinen Formen von 3-[(2-{[4-(Hexyloxycarbonylamino-imino-methyl)-phenylamino]-methyl}-1-methyl-1*H-*benzimidazol-5-carbonyl)-pyridin-2-yl-aminol-proplonsäure-ethylester - Methansulfonat.
Figur 2 zeigt die Thermoanalyse und Schmelzpunktbestimmung (DSC) für die drei kristallinen Formen von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Methansulfonat.

## Patentansprüche

1. 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat in kristalliner Form, **gekennzeichnet durch** einen Schmelzpunkt von T_{Smp}= .= 190 ± 3°C (Form II) (bestimmt über DSC; Auswertung über Peak-Maximum; Heizrate: 10°C/min).

2. Arzneimittel, enthaltend das Salz 3-[(2-{[4-(Hexyloxycarbonylamino-imino-methyl)-phenylamino]-methyl}-1-methyl-1*H*-benzimidazol-5-carbonyl)-pyridin-2-ylamino]-propionsäure-ethylester-Methansulfonat gemäß Anspruch 1 neben gegebenenfalls einem oder mehreren inerten Trägerstoffen und/oder Verdünnungsmitteln.

3. Verwendung von 3-[(2-{[4-(Hexyloxycarbonylamino-imino-mothyl)-phenylamino]-methyl}-1-methyl-1*H*-benzimidazol-5-carbonyl)-pyridin-2-yl-amino]-propionsäure-ethylester-Methansulfonat gemäß Anspruch 1 zur Herstellung eines Arzneimittels, welches zur postoperativen Prophylaxe von tiefen Venenthrombosen und zur Prophylaxe von Schlaganfällen geeignet ist.

4. Verfahren zur Herstellung eines Arzneimittels gemäß Anspruch 2, **dadurch gekennzeichnet, dass** auf nichtchemischem Wege das Salz 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat gemäß Anspruch 1 in einen oder mehrere inerte Trägerstoffe und/oder Verdünnungsmittel eingearbeitet wird.

5. Verfahren zur Herstellung von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat Polymorph II gemäß Anspruch 1, **dadurch gekennzeichnet, daß** man
a) eine Lösung von 3-[(2-{[4-(Hexyloxycarbonylamino-imino-methyl)-phenylamino]-methyl}-1-methyl-1*H-*benzimidazol-5-carbonyl)-pyridin-2-yl-amino]-propionsäure-ethylester in Aceton bei einer Temperatur von ca. 40 °C bis 46 °C langsam mit einer Lösung von einem geringen Unterschuß Methansulfonsäure in Aceton versetzt,
b) bei einer Temperatur von ca. 40 °C bis 46 °C etwa 1 Stunde nachrührt,
c) auf ca. 17 °C bis 23 °C abkühlt und weitere 40 bis 80 Minuten lang bei dieser Temperatur nachrührt,
d) die ausgefallenen Kristalle von 3-[(2-{[4-(Hexyfoxycarbonylamino-imino-methyl)-phenylamino]-methyl}-1-methyl-1*H*-benzimidazol-5-carbonyl)-pyridin-2-yl-amino]-propionsäure-ethylester-Methansulfonat Form II absaugt und
e) das so erhaltene Produkt mindestens 4 Stunden bei maximal 50 °C im Vakuum trocknet.

6. Verfahren zur Herstellung von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-eihylester-Methansulfonat Polymorph II gemäß Anspruch 1, **dadurch gekennzeichnet, daß** man
a) eine Suspension von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat Polymorph I in Aceton unter Rühren ca. 4 Stunden lang auf 45 °C bis 50 °C erwärmt,
b) dann auf ca. 15 °C abkühlt,
c) die ausgefallenen Kristalle von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat Form II absaugt und
d) das so erhaltene Produkt mindestens 4 Stunden bei maximal 50 °C im Vakuum trocknet.

7. Verfahren zur Herstellung von 3-[(2-{[4-(Hexyloxycarbonylamino-imino-methyl)-phenylamino]-methyl}-methyl-1*H*-benzimidazol-5-carbonyl)-pyridin-2-yl-amino]-propionsäure-ethylester-Methansulfonat Polymorph II gemäß Anspruch 1, **dadurch gekennzeichnet, daß** man
a) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat Polymorph I in Aceton vorlegt und
b) die so erhalten Mischung unter Rühren mindestens eine Stunde lang auf 40 °C bis 46 °C erwärmt,
c) dann auf ca. 17 °C bis 23 °C abkühlt und weitere 40 bis 80 Minuten lang bei dieser Temperatur nachrührt,
d) die ausgefallenen Kristalle von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat Form II abtrennt und
e) das so erhaltene Produkt mindestens 4 Stunden bei maximal 50 °C im Vakuum trocknet.

## Claims

1. Ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate-methanesulphonate in crystalline form, **characterised by** a melting point of T_{m.p..} = 190 ± 3°C (form II) (determined by DSC; evaluation by peak maximum; heating rate: 10°C/min).

2. Pharmaceutical composition, containing the salt ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate-methanesulphonate according to claim 1, optionally together with one or more inert carriers and/or diluents.

3. Use of ethyl 3-[(2-([4-(hexyloxycarbonylamino-imino-methyl)-phenylamino]-methyl}-1-methyl-1H-benzimidazole-5-carbonyl)-pyridin-2-ylamino]-propionate-methanesulphonate according to claim 1 for preparing a pharmaceutical composition which is suitable for the post-operative prophylaxis of deep vein thrombosis and the prevention of stroke.

4. Process for preparing a pharmaceutical composition according to claim 2, **characterised in that** by a non-chemical method the salt ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate-methanesulphonate according to claim 1 is incorporated in one or more inert carriers and/or diluents.

5. Process for preparing ethyl 3-[(2-{[4-(hexyloxycarbonylamino-iminomethyl)-phenylamino]-methyl}-1-methyl-1H-benzimidazole-5-carbonyl)-pyridin-2-yl-amino]-propionate-methanesulphonate polymorph II according to claim 1, **characterised in that**
a) a solution of a slight deficiency of methanesulphonic acid in acetone is slowly added to a solution of ethyl 3-[(2-{[4-(hexyloxycarbonylamino-iminomethyl)-phenylamino]-methyl}-1-methyl-1H-benzimidazole-5-carbonyl)-pyridin-2-yl-amino]-propionate in acetone at a temperature of approx. 40°C to 46°C,
b) the mixture is stirred for about 1 hour at a temperature of approx. 40°C to 46°C,
c) cooled to approx. 17°C to 23°C and stirred for a further 40 to 80 minutes at this temperature,
d) the precipitated crystals of ethyl 3-[(2-{[4-(hexyloxycarbonylamino-iminomethyl)-phenylamino]-methyl}-1-methyl-1H-benzimidazole-5-carbonyl)-pyridin-2-yl-amino]-propionate-methanesulphonate form II are suction filtered and
e) the product thus obtained is dried *in vacuo* for at least 4 hours at a maximum of 50°C.

6. Process for preparing ethyl 3-[(2-{[4-(hexyloxycarbonylamino-iminomethyl)-phenylamino]-methyl}-1-methyl-1H-benzimidazole-5-carbonyl)-pyridin-2-yl-amino]-propionate-methanesulphonate polymorph II according to claim 1, **characterised in that**
a) a suspension of ethyl 3-[(2-([4-(hexyloxycarbonylamino-imino-methyl)-phenylamino]-methyl}-1-methyl-1H-benzimidazole-5-carbonyl)-pyridin-2-ylamino]-propionate-methanesulphonate polymorph I in acetone is heated to 45°C to 50°C for approx. 4 hours with stirring,
b) then cooled to approx. 15°C,
c) the precipitated crystals of ethyl 3-[(2-{[4-(hexyloxycarbonylamino-iminomethyl)-phenylamino]-methyl}-1-methyl-1H-benzimidazole-5-carbonyl)-pyridin-2-yl-amino]-propionate-methanesulphonate form II are suction filtered and
d) the product thus obtained is dried in vacuo for at least 4 hours at a maximum 50°C.

7. Process for preparing ethyl 3-[(2-{[4-(hexyloxycarbonylamino-iminomethyl)-phenylamino]-methyl}-1-methyl-1H-benzimidazole-carbonyl)-pyridin-2-yl-amino]-propionate-methanesulphonate polymorph II according to claim 1, **characterised in that**
a) ethyl 3-[(2-([4-(hexyloxycarbonylamino-imino-methyl)-phenylamino]-methyl}-1-methyl-1H-benzimidazole-5-carbonyl)-pyridin-2-yl-amino]-propionate-methanesulphonate polymorph I is placed in acetone and
b) the mixture thus obtained is heated to 40°C to 46°C for at least one hour with stirring,
c) then cooled to approx. 17°C to 23°C and stirred for a further 40 to 80 minutes at this temperature,
d) the precipitated crystals of ethyl 3-[(2-{[4-(hexyloxycarbonylamino-iminomethyl)-phenylamino]-methyl}-1-methyl-1H-benzimidazole-5-carbonyl)-pyridin-2-yl-amino]-propionate-methanesulphonate form II are separated off and
e) the product thus obtained is dried in vacuo for at least 4 hours at a maximum 50°C.

## Revendications

1. Méthanesulfonate d'éthylester d'acide 3-[(2-{[4-(hexyloxycarbonylamino-imino-méthyl)-phénylamino]-méthyl}-1-méthyl-1*H*-benzimidazol-5-carbonyl)-pyridin-2-yl-amino]-propionique sous forme cristalline, **caractérisé par** un point de fusion de T_{Smp.} = 190 ± 3°C (forme II) (déterminé par DSC ; évaluation via le maximum du pic ; vitesse de chauffage : 10°C/min).

2. Médicament contenant le sel méthanesulfonate d'éthylester d'acide 3-[(2-{[4-(hexyloxycarbonylamino-imino-méthyl)-phénylamino]-méthyl}-1-méthyl-1*H*-benzimidazol-5-carbonyl)-pyridin-2-yl-amino]-propionique selon la revendication 1 outre éventuellement un ou plusieurs vecteurs et/ou diluants inertes.

3. Utilisation du méthanesulfonate d'éthylester d'acide 3-[(2-{[4-(hexyloxycarbonylamino-imino-méthyl)-phénylamino]-méthyl}-1-méthyl-1*H-*benzimidazol-5-carbonyl)-pyridin-2-yl-amino]-propionique selon la revendication 1 pour la production d'un médicament qui convient pour la prophylaxie postopératoire des thromboses veineuses profondes et pour la prophylaxie des attaques.

4. Procédé de production d'un médicament selon la revendication 2 **caractérisé en ce que**, par voie non chimique, le sel méthanesulfonate d'éthylester d'acide 3-[(2-{[4-(hexyloxycarbonylamino-imino-méthyl)-phénylamino]-méthyl}-1-méthyl-1*H*-benzimidazol-5-carbonyl)-pyridin-2-yl-amino]-propionique selon la revendication 1 est incorporé dans un ou plusieurs vecteurs et/ou diluants inertes.

5. Procédé de production de méthanesulfonate d'éthylester d'acide 3-[(2- {[4-(hexyloxy-carbonylamino-imino-méthyl)-phénylamino]-méthyl}-1-méthyl-1*H*-benzimidazol-5-carbonyl)-pyridin-2-yl-amino]-propionique polymorphe II selon la revendication 1 **caractérisé en ce que**
a) à une solution de éthylester d'acide 3-[(2-{[4-(hexyloxy-carbonylamino-imino-méthyl)-phénylamino]-méthyl}-1-méthyl-1*H*-benzimidazol-5-carbonyl)-pyridin-2-yl-amino]-propionique dans l'acétone on ajoute lentement à une température d'environ 40°C à 46°C une solution d'un faible défaut d'acide méthanesulfonique dans l'acétone,
b) on agite pendant environ 1 heure à une température d'environ 40°C à 46°C,
c) on refroidit à environ 17°C à 23°C et on agite à cette température pendant 40 à 80 minutes supplémentaires,
d) un essore les cristaux de méthanesulfonate d'éthylester d'acide 3-[(2-{[4-(hexyloxy-carbonylamino-imino-méthyl)-phénylamino]-méthyl}-1-méthyl-1*H-*benzimidazol-5-carbonyl)-pyridin-2-yl-amino]-propionique forme II précipités et
e) on sèche sous vide le produit ainsi obtenu pendant au moins 4 heures à 50°C au maximum.

6. Procédé de production de méthanesulfonate d'éthylester d'acide 3-[(2-{[4-(hexyloxy-carbonylamino-imino-méthyl)-phénylamino]-méthyl}-1-méthyl-1*H*-benzimidazol-5-carbonyl)-pyridin-2-yl-amino]-propionique polymorphe II selon la revendication 1 **caractérisé en ce que**
a) on chauffe une suspension de méthanesulfonate d'éthylester d'acide 3-[(2-{[4-(hexyloxy-carbonylamino-imino-méthyl)-phénylamino]-méthyl}-1-méthyl-1*H-*benzimidazol-5-carbonyl)-pyridin-2-yl-amino]-propionique polymorphe I dans l'acétone sous agitation pendant environ 4 heures à 45°C à 50°C,
b) on refroidit ensuite à environ 15°C,
c) on essore les cristaux de méthanesulfonate d'éthylester d'acide 3-[(2-{[4-(hexyloxy-carbonylamino-imino-méthyl)-phénylamino]-méthyl}-1-méthyl-1*H-*benzimidazol-5-carbonyl)-pyridin-2-yl-amino]-propionique forme II précipités et
d) on sèche sous vide le produit ainsi obtenu pendant au moins 4 heures à 50°C au maximum.

7. Procédé de production de méthanesulfonate d'éthylester d'acide 3-[(2-{[4-(hexyloxy-carbonylamino-imino-méthyl)-phénylamino]-méthyl}-1-méthyl-1*H*-benzimidazol-5-carbonyl)-pyridin-2-yl-amino]-propionique polymorphe II selon la revendication 1 **caractérisé en ce que**
a) un dispose au préalable méthanesulfonate d'éthylester d'acide 3-[(2-{[4-(hexyloxy-carbonylamino-imino-méthyl)-phénylamino]-méthyl}-1-méthyl-1*H-*benzimidazol-5-carbonyl)-pyridin-2-yl-amino]-propionique polymorphe I dans l'acétone et
b) on chauffe le mélange ainsi obtenu sous agitation pendant au moins une heure à 40°C à 46°C,
c) puis on refroidit à environ 17°C à 23°C et on agite à cette température pendant 40 à 80 minutes supplémentaires,
d) on sépare les cristaux de méthanesulfonate d'éthylester d'acide 3-[(2-{[4-(hexyloxy-carbonylamino-imino-méthyl)-phénylamino]-méthyl}-1-méthyl-1*H-*benzimidazol-5-carbonyl)-pyridin-2-yl-amino]-propionzque forme II précipités et d) on sèche sous vide le produit ainsi obtenu pendant au moins 4 heures à 50°C au maximum.
